Europäisches Patentamt

European Patent Office

Office européen des brevets

(19)

(11) **EP 1 201 680 A2**

(12) **EUROPEAN PATENT APPLICATION**

(43) Date of publication:
**02.05.2002 Bulletin 2002/18**

(21) Application number: **01400715.7**

(22) Date of filing: **20.03.2001**

(51) Int Cl.⁷: **C07K 9/00**, C07K 14/47,
C12N 15/11, C12N 15/62,
C12N 15/63, A61K 38/00,
A61K 48/00, A61P 35/00

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **24.10.2000 US 242680**

(71) Applicant: **Urogene Société anonyme
91000 Evry (FR)**

(72) Inventors:
• **Latil, Alain
92370 Chaville (FR)**
• **Berthon, Philippe
77930 Perthes en Gatinais (FR)**
• **Cussenot, Olivier
75020 Paris (FR)**

(74) Representative: **Sauvage, Renée
Cabinet Sauvage
65, Boulevard Soult
75012 Paris (FR)**

(54) **Tumor suppressor polypeptide issued from CHC1-L (chromosome condensation 1-like)**

(57) The present invention relates to a new polypeptide useful for the treatment of metastatic disease, and more particularly of prostate cancer.

This polypeptide is of about 15 to about 30 amino acids, and corresponds to the protein fragment which is encoded by the 5' end of the tumor suppressor gene CHC1-L.

The present invention also relates to a nucleotide sequence encoding for said polypeptide and vector, host cell and pharmaceutical composition including said polypeptide or nucleotide sequence.

It is also an object of the present invention to develop a new method, and the corresponding kit, for the prostate *in vitro* diagnosis.

EP 1 201 680 A2

**Description**

The present invention relates to methods for the diagnosis, the evaluation and the treatment of metastatic diseases, specifically of prostate cancer. More particularly, the present invention relates to a new polypeptide, acting as a tumor suppressor gene.

Prostate cancer is one of the most common malignancies diagnosed among men in Western countries (1) [the bold numbers in brackets refer to the enclosed reference list].

To date, many therapeutic regimens for treating prostate tumors have been developed, including external radiation therapy, Curietherapy, surgery, radical prostatectomy, and the like as well as combinations of two or more of the above.

Nevertheless, none of these regimens is totally satisfactory as, for the former, radiation induces mutations in the organism and in the prostate and, for the latter, surgery may lead to sterility and compromise the quality of life of the patient. For these reasons, many studies have been done to understand the exact molecular mechanisms underlying the onset and progression of prostate cancer.

The genetics of cancer is complex, involving multiple dominant, positive regulators of the transformed state (oncogenes) as well as multiple recessive, negative regulators (tumor suppressor genes). Over one hundred oncogenes have been characterized. The number of tumor suppressor genes is expected to increase beyond fifty.

Several tumor suppressor genes have been identified. Examples include the Rb gene, which is involved in retinoblastoma and osteosarcoma ; p53, which is involved in osteosarcoma and adrenocortical, breast and brain cancers ; WT-1, which is involved in Wilm's tumor, nephroblastoma and neurofibromatosis ; adenomatous polyposis coli (APC), which is involved in adenomatous polyposis ; and deleted colorectal cancer (DCC), which is involved with a somatic mutation in colon. Like other neoplastic diseases, prostate cancer originates and develops by accumulating genetic alterations or mutations in one or more genes. Such mutations which result in cancer formation can be in proto-oncogenes or in tumor suppressor genes. Mutations in tumor suppressor genes result in loss of function of these genes, i.e. in the negative regulation of tumor cells' growth.

Recently, it has been discovered that the most frequent type of tumor-associated mutation is somatic loss of heterozygosity (LOH) in distinct chromosomal regions (**2**), and one of the more recurrent aberrations observed in prostate tumors involves chromosome arm 13q (**3, 4**). For LOH, the remaining allele is presumed to be nonfunctional, either because of a preexisting inherited mutation, or because of a secondary sporadic mutation.

A smallest common deleted region on chromosome 13q14.3 in primary prostate tumors has been found (**5-9**), pointing to a gene with an important role in the initiation and/or progression of prostatic carcinoma. Moreover, evidence for allele loss at the 13q14.3 locus has also been found in other cancers, including breast cancer, ovarian carcinoma, head and neck squamous cell carcinoma and pituitary tumors (**10-13**).

LOH was initially suspected to affect the retinoblastoma susceptibility gene (RB1) located in the commonly deleted region. However, no correlation between LOH at the RB1 locus and the decreased expression of this gene has been obtained, indicating that RB1 is not the main target of the observed LOH at 13q14.3 (**3, 7, 14**).

A new gene, called CHC1-L for chromosome condensation 1-like, has recently been mapped close to RB1, making it a candidate (**15**). It has been reported that this new gene shows significant homology with the seven intradomain repeats of the regulator of chromosome condensation (RCC1) gene, which acts as a guanine nucleotide exchange factor (GEF) for the Ras-related GTPase Ran (**16, 17**).

The structural analysis of CHC1-L has shown that it is composed of 14 exons, including 2 (exons 2 and 3) that are alternatively used together, separately or skipped and generate 4 possible mRNA isoforms. The first in-frame ATG is located in exon 3. As a consequence, CHC1-L full-length product and the Δ2-CHC1-L variant encode an isoform of 551 amino acids, called long form (L), whereas Δ3 and Δ2/3-CHC1-L, in which exon 3 is absent, encode an isoform of 526 amino acids, called short form (S) using an ATG in exon 4 (15).

To date, no supplementary studies have been performed to confirm that CHC1-L is a putative tumor suppressor gene.

The present invention relates, not only to the revelation that CHC1-L is actually the tumor suppressor gene, but also to the identification of a polypeptide, issued from the 5' end of CHC1-l, which is of interest for prostate cancer diagnosis and treatment.

First, in order to confirm the CHC1-L anti-tumor function, the effect of the LOH at 13q14.3 locus on CHC1-L expression in prostate tissues has been assessed. To address this issue, several prostate tumors as well as prostate tumor cell lines (LNCaP, DU145 and PC3) have been use to correlate LOH status at 13q14.3 using 3 polymorphic markers with CHC1-L expression. Also, in less aggressive epithelial prostatic cell lines (PNT2, PNT1A and PNT1B), CHC1-L levels of expression were leveled, using a real-time quantitative RT-PCR assay.

The detailed description of all these experiments are among the examples presented.

The results show that CHC1-L is underexpressed in most of the tested tumors, and especially CHC1-L is down-regulated in most of the prostate tumors with a 13q14.3 deletion, suggesting that abnormal CHC1-L mRNA expression is likely to result from deletion of the region.

So, there is a trend toward an association between LOH status at 13q14.3 and CHC1-L underexpression in prostate tumors.

The three above mentioned prostate tumor cell lines showed markedly decreased expression of CHC1-L compared to prostatic epithelial cell lines. It is noteworthy, that losses of chromosome arm 13q have been found in the three prostate tumor cell lines by means of comparative genomic hybridization. Until now, it has been supposed that the RB1 gene, located in the smallest common deleted region, is involved in prostate tumorigeneis, but no correlation between LOH at 13q14.3 and decreased expression of this gene could be made. Although its role in prostate carcinogenesis remains to be elucidated, data are consistent with the fact that RB1 gene is not the main target of the observed LOH.

Compared to the negative results obtained with the gene RB1, these results support the idea that CHC1-L is the candidate gene of the observed LOH at 13q14.3.

Unexpectedly, it has been shown that the frequency of the 551 amino acid isoform (L) is lower in tumor than in normal prostate tissue, suggesting that the disappearance of this form could be associated with prostate tumorigenesis. Regarding these results, each CHC1-L variant has been quantified by means of a real-time PCR.

The obtained data indicate that decreases in total CHC1-L expression observed in prostate tumor cell lines is related to a decreased expression of the wild type isoform, i.e. the 551 amino acid isoform (L).

Hence, the results obtained reveal that the ratio between 551aa / 526aa isoforms, i.e. L/S, is higher in prostatic epithelial cell lines than in prostate tumor cell lines.

From these results, the inventors have identified a new polypeptide, corresponding to the difference between the two isoforms, which is involved in the prostate tumorigenesis, and may be used in therapeutic and diagnostic applications.

The present invention relates to such studies, and more particularly to the unexpected identification of a new polypeptide, which is encoded by a part of the 5' end of the CHC1-L gene.

More particularly, the present invention concerns a polypeptide, of about 15 to about 30 amino acids, which corresponds to the protein fragment encoded by the 5' end of the tumor supressor gene CHC1-L.

In a preferred embodiment of the present application, a preferred polypeptide consists in the whole, or a part of the, sequence SEQ ID NO : 1.

By "polypeptide", is meant any peptide or protein comprising two or more amino acids joined to each other by peptide bonds or modified peptide bonds. "Polypeptide" refers to both (1) short chains, commonly referred to as peptide, oligopeptide and oligomer and to (2) longer chains, generally referred to as protein. Polypeptides include those modified either by natural processes, such as processing and other post-translational modifications, but also by chemical modification techniques, well known to those skilled in the art. Modifications can occur anywhere in a polypeptide, including the polypeptide backbone, the amino acid side-chains, and the amino or carboxyl termini. Modifications include, for example, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine dimers, formation of pyroglutamate, formylation, gamma-carboxylation, glycosylation, GPT anchor formation, hydroxylation, iodination, methylation, myristoylation, oxidation, proteolytic processing, phosphorylation, prenylation, racemization, glycoylation, lipid attachment, sulfation, gamma-carboxylation of glutamic acid residues, hydroxylation and ADP-ribosylation, selenoylation, sulfation, transfer-RNA mediated addition of amino acids to proteins, such as arginylation, and ubiquitination.

The polypeptide of this invention is preferably made by recombinant genetic engineering techniques, which are well known by the man skill in the art.

More particularly, in a preferred embodiment, the polypeptide of the present invention can show one or more conservative(s) substitution(s) and/or addition(s) and/or deletion(s) of one or more amino acid(s).

By "conservative substitution", is meant any replacement of one or more amino acid(s) by another (others) without causing any loss of function to the polypeptide.

By "conservative deletion", is meant any suppression of one or more amino acid(s) without causing any loss of function to the polypeptide.

By "conservative addition", is meant any addition of one or more amino acid(s) without causing any loss of function to the polypeptide.

Such modifications can naturally appear, due to, for example, a modification of the environment, or can be the result of an artificial manipulation.

More particularly, the polypeptide according to the present invention can be substituted at its N-terminal and/or C-terminal end(s).

In a preferred embodiment, the substituant is an active principle selected in the group consisting in a growth-factor, a neuropeptide, a transcription factor, a signal molecule, a repressor or antagonist of transcription or replication, a toxin, an antitoxin or an apoptosis inducer.

By "active principle", is meant any product of interest, i.e. biological, pharmaceutical or diagnostic, marker product. More particularly, it can consist in a protein, such as a hormone, a cytokine, an apolipoproteine, a growth-factor, a neuropeptide, a transcription factor, a signal molecule, a repressor or antagonist of transcription or replication, a toxin, an antitoxin, an antibiotic, or an apoptosis inducer.

Another aspect of the present invention relates to a nucleotidic sequence encoding for a polypeptide according as previously described.

By "nucleotidic sequence", is meant a sequence of DNA or RNA.

The term "encoding" refers to an inherent property of a sequence of nucleotides in a nucleic acid, such as a gene in a chromosome or an mRNA, to serve as a template for synthesis of other polymers and macromolecules in biological processes having a defined sequence of nucleotides (rRNA, tRNA or other RNA molecules) or amino acids (peptides and proteins).

In another embodiment of the present invention, the nucleotide sequence shows the whole, or a part of the, sequence SEQ ID NO : 2.

A further aspect of the invention relates to the use of such nucleotidic sequences for the preparation of vectors. According to a preferred embodiment, the present invention relates to a recombinant vector which comprises, or includes, at least the nucleotide sequence as previously described.

By "vector", is meant any viral or non-viral expression system which is known by the man skilled in the art.

The expression "recombinant" refers to the result of methods, reagents, and laboratory manipulations in which nucleic acids or other biological molecules are enzymatically, chemically or biologically cleaved, synthesized, combined or otherwise manipulated *in vitro* to produce desired products in cells or other biological systems.

In a preferred embodiment of the present invention, the vector consists in a viral vector.

Such viral vectors which are preferred are selected from adenovirus, adeno associated virus (AAV), retrovirus and lentivirus.

In another preferred embodiment of the present invention, the recombinant vector consists in a non viral vector.

Such non-viral vectors which are preferred are selected from an expression and non-expression plasmid, i. e. an autonomous self-replicating circular DNA, possibly covered with a synthetic polymer, or polysaccharide, or protein, or peptide fragment or mixture of two or more of the above, where the protein is an antibody.

More particularly, numerous known recombinant vectors can be used, such as pBR322, pACYC177, pKT230, pGV1106, pLAFR1, pME290, pHV14, pIJ61, pUC6, Yip5, baculovirus cell system, Ycp19, pSV2neo or pCDNA1neo.

In a preferred embodiment, the vector according to the present invention consists in a plasmid possibly covered with a synthetic polymer or a polysaccharide, a protein, a peptide fragment or a mixture of two or more of the above.

The present invention also relates to a transfected host cell which includes a nucleotide sequence and/or a vector as previously described.

More particularly, transfected cell refers to a cell that comprises a recombinant nucleic acid molecule, typically a recombinant plasmid or other expression vector as previously described.

Such a transfected cell may be a prokaryotic or eukaryotic cell, including a bacterial, yeast, insect or mammalian cell.

An interest of such transfected, or recombinant, cells is that they can express genes that are not found within the native (non-transfected) form of the cell. For example, such cells can produce a "recombinant protein", such as the polypeptide according to the present invention, which refers to a protein that is produced by expression of a recombinant DNA that encodes the amino acid sequence of the protein.

A further aspect of the present invention relates to the use of a polypeptide and/or a nucleotide sequence and/or a vector as previously described for the preparation of a pharmaceutical composition.

By "pharmaceutical composition", is meant a composition suitable for pharmaceutical or therapeutic use in a mammal.

The pharmaceutical compositions comprise a pharmacologically effective amount of an active agent, alone or in combination with one or several pharmaceutically acceptable carrier(s).

"Pharmacologically effective amount" refers to that amount of an agent effective in producing the intended pharmacological result.

In the present invention, the active agent consists in the polypeptide and/or nucleotidic sequence and/or recombinant vector previously described.

The active agent can be administrated alone, but will generally be administrated in admixture with a pharmaceutical carrier.

In yet another aspect, the present invention relates to a pharmaceutical composition including, in combination with one or more pharmaceutically acceptable carrier(s), a polypeptide and/or a nucleotide sequence and/or a vector as previously described.

By "pharmaceutically acceptable carrier", is meant a pharmaceutical vehicle, buffer or excipient, such as a phosphate buffered saline solution, 5% aqueous solution of dextrose, and emulsions, such as an oil/water or water/oil emulsion, and various types of wetting agents and/or adjuvants. Preferred pharmaceutical carriers depend upon the intended mode of administration of the active agent.

Typical modes of administration include enteral, oral, parenteral, subcutaneous, intramuscular, intravenous, intraperitoneal, topical, transdermal or transmucosal administration.

The choice of a specific carrier, according to the chosen mode of administration, is effected by reference to criteria which are well known by the man skilled in the art.

For example, for solid compositions, such as tablets, pills, free or encapsulated powders or granules, the active principle can be combined with diluents, such as lactose, dextrose and/or glycine ; lubricant, such as silica or stearic acid ; linkers, such as magnesium or aluminum silicates, gelatin or starch ; or absorbents, colorants, aroma and sweeteners.

In the case of semi-solid compositions, such as suppositories, the carrier can consist in a fatty emulsion or suspension.

Liquid compositions, which can be injectable or integrated in soft capsules or ovules, can be prepared by dissolution, dispersion, etc., of the active principle in a pharmaceutically pure solvent, such as water, saline solution, aqueous dextrose, glycerol, ethanol or analogs.

The pharmaceutical compositions, according to the present invention, can also be sterilized and/or include adjuvants or non-toxic substances such as preserving, emulsificating, wetting, coloring, flavoring or stabilizing agents, agents helping the dissolution, salts for regulating the osmotic pressure and/or others buffers.

Moreover, they can also comprise other therapeutic agents, such as other anti-tumoral substances.

The pharmaceutical composition object of the present invention can include, in combination with one or more pharmaceutically acceptable carrier(s), a factor mimicking the function of the polypeptide and/or nucleotide sequence and/or vector as previously described.

By "factor mimicing", is meant a molecule which replaces CHC1-L action.

Without wishing to be bound by any theory, it is thought that the mimic factor, in said pharmaceutical compositions, can act, as a tumor suppressor, in several ways.

Firstly, the mimic factor of said pharmaceutical composition can act as a blocking factor of the activation of the genes normally inhibited by CHC1-L.

By "blocking factor of the activation", is meant any molecule that prevents attaining or assuming the active form.

For example, such a mimic factor can be selected from a "half-receptor" or a "half-signal" peptide.

In another embodiment, the mimic factor of said pharmaceutical composition can act as a blocking factor of the expression of the genes normally inhibited by CHC1-L.

By "blocking factor of the expression", is meant any molecule that blocks the synthesis of the RNA template corresponding to the gene.

For example, such a mimic factor can consist in a stable antisense DNA.

In another embodiment, the mimic factor of said pharmaceutical composition can act as a blocking factor of the gene penetration normally inhibited by CHC1-L.

By "blocking factor of penetration", is meant any molecule which blocks the penetration of CHC1-L into its active site, or from reaching the cell compartment, i.e. membrane, nucleus, cytoplasm or extracellular space, where it is active.

For example, such a mimic factor can consist in a chemical and/or a natural product, such as a product selected from a peptide, a polysaccharide, a nucleic acid or an antibody.

Another application of the present invention can consist in the use of the polypeptide and/or nucleotidic sequence and/or vector according to the invention for cellular addressing.

More particularly, any anti-tumoral substances can be coupled with the polypeptide and/or nucleotidic sequence and/or vector according to the invention, which will be directly addressed to the concerned region of the gene.

Still another application of the present invention can consist in the use of the polypeptide and/or nucleotidic sequence and/or vector according to the invention for the screening and the identification of any factors, genes, receptors, ligands or other elements playing a role in the CHC1-L kinetic.

Such studies will allow the use of any antitumoral substances, and will address and/or affix them directly to the identified elements for blocking cancer development. Antitumoral substances can consist in, for example, growth and/or replication inhibitors, as well as apoptosis inducers including antibodies, e.g. Herceptin, which is an anti Her2/NEU).

In a preferred embodiment, the present invention, relates a pharmaceutical composition for the cancer treatment.

More particularly, the present invention relates to a pharmaceutical composition for the prostate cancer treatment.

In practical use, such pharmaceutical compositions are administrated to a patient in therapeutically effective amount, or dose, prescribed by a doctor or a veterinarian.

The expression "therapeutically effective amount" refers to an amount of pharmaceutical compositions that is administrated to a patient suffering from a disease and is sufficient to cure or at least partially arrest or ameliorate the symptoms of the disease and its complications. The amount will depend on the severity of the disease and on the patient and can be determined by standard pharmaceutical procedures in cell cultures or experimental animals. The therapeutic index is the ratio LD50/ED50 (ED50 is the effective dose in 50% of the population ; LD50 is the lethal dose for 50% of the population), and pharmaceutical compositions that exhibit large therapeutic indices are preferred. The data obtained from cell cultures or animal studies is used in formulating a range of circulating concentrations that include the ED50 with little or no toxicity. The dosage varies within this range depending upon the dosage form employed, sensitivity of the patient and the route of administration.

A further aspect of the invention relates to the use of a polypeptide and/or a nucleotide sequence and/or a vector, as previously described, as an *in vitro* diagnostic agent.

By "diagnostic agent", is meant any marker or combination of markers that indicate and monitor disease status.

Regarding the obtained data concerning the levels of RNA encoding for each form of CHC1-L, previously related, the inventors have defined a ratio between each forms (L) and (S), and have evaluate this ratio in safe and tumoral cells.

In a preferred embodiment, the present invention relates to a method for the *in vitro* diagnosis of the prostate cancer consisting in evaluating the RNA level of the peptide as previously described by measuring the ratio between the long form (L) and the short form (S) of CHC1-L.

This evaluation has revealed that the ratio L/S is, in normal cells, of the order of 3, whereas it is, in tumoral cells, close to 2.

Hence, the present invention relates to a method which consists in :

a) measuring the level of RNA encoding for the long form (L) of CHC1-L,
b) measuring the level of RNA encoding for the short form (S) of CHC1-L,
c) determining the ratio L/S, and
d) declaring the diagnosis positive for cancer if the obtained ratio of step c) is close to 2.

The expression "close to 2" means, by comparison with the normal value of about 3, which is comprised between about 1 and about 2,5.

A further aspect of the present invention relates to a kit for the implementation of the above process including :

a) primer(s) for the amplification of the RNA encoding for the two forms (L) and (S) of CHC1-L,
b) polymorphic markers,
c) primer(s) for the PCR amplification of the polymorphic markers, and
d) buffers and enzymes for the amplification, marking and hybridization reactions.

Polymorphic markers can be, for example, selected from the group consisting in microsatellites, SNP or RFLP markers, as well as D13S272, D13S284 or D13S153.

Primers for the amplification of these polymorphic markers are known by the man skilled in the art (**22**).

Primers for the RNA amplification of each form of CHC1-L are chosen in the list referenced in table 1.

The present invention will be better understood from the following description of the experiments referring to the enclosed figures, wherein :

Figure 1 shows the relative expression levels of CHC1-L in 26 clinically prostate adenocarcinoma samples (light gray bars), 10 hormone refractory prostate tumors (dark gray bars), 6 matched normal prostate tissues and RNA from a pool of 47 normal human prostate tissues (Clontech, Palo, Alto, CA) (black bars),
Figure 2 shows the relative expression levels of CHC1-L in 3 prostatic epithelial cell lines (PNT2, PNT1A, and PNT1B) (black bars) and 3 prostate tumor cell lines (LNCaP, DU145, and PC3) (light gray bars), and
Figure 3 shows the relative expression levels of CHC1-L variants in normal and neoplastic prostate tissues.

## MATERIALS AND METHODS

### I. Patients and Samples

Thirty-six primary prostate tumors and 6 prostate cell lines (PNT2, PNT1A, PNT1B, LNCaP, DU145, and PC3) were analyzed. Tumor specimens were obtained from patients undergoing surgery at St-Louis Hospital in Paris, La Cavale Blanche Hospital in Brest, and the CHU in Nancy (France). Twenty-six patients had clinically localized prostate tumors and 10 had hormone-refractory recurrent prostate carcinomas.

Prostate samples were obtained in two ways as followed:

Clinically localized prostate tumors were obtained by radical prostatectomy. Specimens were first sliced thickly, and samples were cut from suspect areas. Part of the selected tissue was immediately placed in liquid nitrogen for RNA extraction, while adjacent sections were stained with H/E (hematoxylin and eosine) to determine the proportion of tumor cells in each sample.

Patients with metastatic disease at diagnosis, where radical surgery is excluded, have been treated by endocrine therapy, either by classical androgen deprivation [orchiectomy or luteinizing hormone-releasing hormone (LHRH) agonist] or by maximal androgen blockade (castration combined with antiandrogen). After varying times, these patients relapse and their tumors become clinically androgen independent. These hormone-refractory recurrent prostate carcinomas were obtained by transurethral resection and 6 to 12 chips were obtained during resection.

## II. Selected tissues

Malignant areas from tumor samples were carefully selected by means of microdissection to obtain a homogeneous cell population and thereby avoid "dilution" of tumor-specific genetic changes with nucleic acids from normal and reactive cells present in the same specimen. For these reasons a sample was considered suitable for molecular studies if the proportion of tumor cells exceeded 90% of epithelial cells.

The histological diagnosis, clinical staging based on the TNM system and Gleason score (**18, 19**) were determined in each case during a routine clinical work-up after surgery. Ten were hormone refractory tumors whereas after pathological examination thirteen of the 26 clinically localized tumors were pT2 and 13 were pT3. The Gleason scores of the carcinomas were 4-6 (4 cases), 7 (20 cases) and 8-10 (12 cases).

Six well-characterized normal prostate tissue specimens and RNA from a pool of 47 normal human prostate tissues (Clontech, Palo, Alto, CA) were used to assess basal levels of CHC1-L mRNA in normal prostate tissue. Normal prostate tissues were obtained from 6 of the 26 patients who underwent a radical prostatectomy. Samples were histologically checked and selected for the absence of cancer cells and in respect to their cell type component; indeed, in order to avoid benign prostatic hyperplasia, samples with a majority of stroma cells were excluded from this study.

The three prostatic epithelial cell lines (PNT2, PNT1A, and PNT1B) have been described previously (**18**), and the three prostate tumor cell lines (LNCaP, DU145, and PC3) were obtained from the American Tissue Type Culture Collection (**20**).

Peripheral blood leukocytes or matched normal prostate tissue specimens were used as a source of normal DNA for each patient.

## III. Nucleic acids extraction

### III.1 *RNA extraction* and *cDNA synthesis*

Total RNA were extracted from tissue specimens by using the acid-phenol guanidium method (**21**). The quality of the RNA samples was determined by electrophoresis through agarose gels and staining with ethidium bromide. The 18S and 28S RNA bands were visualized under ultraviolet light. RNA was reverse transcribed in a final volume of 20 $\mu$l containing 1X RT buffer (500 mM each dNTP, 3 mM MgCl$_2$, 75 mM KCl, 50 mM Tris-HCl pH 8.3), 10 units of RNasin™ ribonuclease inhibitor (Promega, Madison, WI), 10 mM dithiothreitol, 50 units of Superscript II RNase H$^-$ reverse transcriptase (Gibco BRL, Gaithersburg, MD), 1.5 mM random hexamers (Pharmacia, Uppsala, Sweden) and 1 $\mu$g of total RNA. The samples were incubated at 20°C for 10 min and 42°C for 30 min, and reverse transcriptase was inactivated by heating at 99°C for 5 min and cooling at 5°C for 5 min.

### III.2 *DNA extraction*

DNA was extracted from the same cells used for the RNA analysis. High-molecular-weight DNA was prepared by proteinase K digestion and phenol/chloroform extraction from tumor specimens and either matched normal tissues or blood samples.

## IV. DNA analysis

Polymorphic markers D13S272, D13S284, and D13S153 (**22**), were used for LOH analysis. The PCR reactions were carried out in a total volume of 15 $\mu$l containing 50 ng of genomic DNA, 3 pmol of each primer, 1 mM for each deoxynucleotide triphosphate, 1.5 mM MgCl2, 10 mM Tris-HCl pH 8.3, 50 mM KCl, and 0.6 units of Taq DNA polymerase. Conditions for amplification were: 94°C for 5 min, 12 cycles of 94°C for 15 sec, 55°C for 15 sec, 72°C for 30 sec followed by 22 cycles of 89°C for 15 sec, 55°C for 15 sec, and 72°C for 30 sec. The final extension step at 72°C was extended to 10 min.

The sensitive Applied Biosystems model 377 DNA sequencing system (Perkin Elmer, Foster City, CA) was used for LOH analyses. Dilutions of PCR products were optimized for each microsatellite and 1 μl aliquots of each amplified product were added to 3.5 μl of deionized formamide containing 0.3 ml of a molecular size marker (Genescan 500 ROX). The mixtures were heat-denatured and 1.5 μl aliquots of each were loaded on 4% polyacrylamide/8M urea gel and run for 3 hours at 1200 V in the sequencer. One of the primer set was fluorescein-labeled at its 5' end by adding Fluorprime during oligonucleotide synthesis and the different fragments were quantified with the Genescan® 672 Fragment Analysis software (Perkin Elmer Applied Biosystems, Foster City, CA), which calculated the size and area of the peaks.

LOH was considered to be present when the relative intensity of the two alleles in tumor DNA differed from the relative intensity in lymphocyte DNA by a factor of at least 1.5; this is a validated cut-off value to determine LOH of microsatellite loci in tumor samples containing >60% cancer cells (**23**). Each analysis was performed at least twice to ensure reproducible detection of LOH (repeat independent PCR amplification, gel separation, and quantification).

<u>V. Real-time RT-PCR</u>

V.1 *Theoretical basis*

Quantitative values are obtained from the threshold cycle (Ct) number at which the increase in signal associated with an exponential growth of PCR product starts to be detected (using PE Biosystems analysis software), according to the manufacturer's manual.

The precise amount of total RNA added to each reaction (based on optical density) and its quality (i.e., lack of extensive degradation) are both difficult to assess. Therefore, transcripts of the gene RPLP0 (also known as 36B4) encoding human acidic ribosomal phosphoprotein P0 as the endogenous RNA control were also quantified, and each sample was normalized on the basis of its RPLP0 content. A prostate tumor (T17) which contained the smallest accurately quantifiable amount of CHC1-L mRNA in the done series was used as calibrator.

Final results, expressed as N-fold differences in CHC1-L expression relative to the RPLP0 gene and the calibrator, termed $N_{CHC1-L}$, were determined as follows:

$$N_{CHC1-L} = 2\ (\Delta Ct_{sample} - \Delta Ct_{calibrtor})$$

where $\Delta Ct$ values of the sample and calibrator are determined by subtracting the average Ct value of the target gene from the average Ct value of the RPLP0 gene. The ratios were normalized such that the mean ratio of the 6 matched normal prostate samples equals a value of 1. Owing to the problem of prostate cellularity, CHC1-L expression in each sample was also assessed according to the proportion of its epithelial cells component. For this purpose, transcripts of Cytokeratin 18 (KRT18), a specific epithelial marker, was also quantified and used as specific endogenous controls. Each sample was, thus, normalized on the basis of its KRT18 content. It is noteworthy that, in each sample, expression levels of KRT18 measured by means of quantitative real time RT-PCR correlated with histo-morphological analysis of the corresponding tissue section.

V.2 *Primers and PCR consumables*

Primers were chosen with the assistance of the computer programs Oligo 4.0 (National Biosciences, Plymouth, MN) and Primer Express (Perkin-Elmer Applied Biosystems, Foster City, CA). The inventors performed BLASTN (**24**) searches against dbEST, htgs and nr (the non redundant set of GenBank, EMBL and DDBJ database sequences) to confirm the total gene specificity of the nucleotide sequences chosen as primers. To avoid amplification of contaminating genomic DNA, one of the two primers was placed at the junction between two exons or in a different exon.

Primer pairs are designated by nucleotide position (relative to CHC1-L GenBank #AF060219 and RPLPO GenBank #NM001002) corresponding to the 5' position followed by the letter U for upper (i.e. sense strand) or L for lower (i.e. antisense strand).

For CHC1-L whole transcript quantification 1451U spans the end of exon 11 and the beginning of exon 12, whereas 1584L lies in exon 12.

D2, D3 and D2/3- CHC1-L variants were quantified using primer pairs 90U/302L, 186U/383L, and 88U/381L, respectively. 90U spans the end of exon 1 and the beginning of exon 3, 186U spans the end of exon 2 and the beginning of exon 4, 88U spans the end of exon 1 and the beginning of exon 4, whereas lower primers (302L, 383L, and 381L) lie in exon 4. Primer sets were, furthermore, checked on PCR reaction for a single band on agarose gel, and their products were purified and sequenced to confirm the specificity.

For the integrity of CHC1-L transcripts, 69U and 393L lie in exon 1 and exon 4, respectively.

The nucleotide sequences of primers are shown in the following Table 1.

EP 1 201 680 A2

TABLE 1

| Gene | oligonucleotides | location | sequence | PCR product size |
|---|---|---|---|---|
| **CHC1-L Quantification** | | | | |
| CHC1-L Whole | 1451 U | Exon 11-12 | 5'-ATAAAGTCCTTCTCAAGATTCGATGT-3' | 134 bp |
| | 1584 L | Exon 12 | 5'-GCTGTCTGTGTATAGGTATTCCAGG-3' | |
| Wild type | 69U | Exon 1 | 5'-AGATGACTGAATACTGGGTTCCAGA-3' | 145 bp |
| | 213L | Exon 2-3 | 5'-GGCAGTCCCTTTGTCAACTTTTC-3' | |
| Δ2 variants | 90 U | Exon 1-3 | 5'-CAGAAATTTAAAACAATCAGGGAC-3' | 117 bp |
| | 302 L | Exon 4 | 5'-ATCTTCAAAGATGACAGAGTAGCC-3' | |
| Δ3 variants | 186 U | Exon 2-4 | 5'-CCAGAGAAAAGTTGACAAAGCCA-3' | 133 bp |
| | 383 L | Exon 4 | 5'-CCAAAGACACAAGCCTGACGA-3' | |
| Δ2/3 variants | 88 U | Exon 1-4 | 5'-TCCAGAAATTTAAAACAATCAGCCA-3' | 133 bp |
| | 381 L | Exon 4 | 5'-AAAGACACAAGCCTGACGAATTAAC-3' | |
| RPLPO | 95 U | Exon 2 | 5'-GGCGACCTGGAAGTCCAACT-3' | 149 bp |
| | 243 L | Exon 3 | 5'-CCATCAGCACCACAGCCTTC-3' | |
| **CHC1-L Integrity*** | 69U | Exon 1 | 5'-AGATGACTGAATACTGGGTTCCAGA-3' | 325 bp |
| | 393L | Exon 4 | 5'-GCCAGCACTGCCAAAGACAC-3' | |

Wild type : full length transcripts

Δ2, Δ3 and Δ2/3 variants : alternatively spliced transcripts in which exon 2, 3 and 2 and 3 is(are) absent, respectively.

\* : The frequency of CHC1-L variants in prostate tissues has been assessed using primer pair 69U/393L.

V.3 *PCR amplification*

All PCR reactions were performed using an ABI Prism 7700 Sequence Detection System (Perkin-Elmer Applied Biosystems). PCR was performed using the SYBR® Green PCR Core Reagents kit (Perkin-Elmer Applied Biosystems). The thermal cycling conditions comprised an initial denaturation step at 95°C for 10 min and 45 cycles at 95°C for 15 sec and 62°C (58°C for D2-CHC1-L variants) for 1 min. Experiments were performed with duplicates for each data point. All samples with a coefficient of variation for Ct value higher than 1% were retested.

VI. Statistical analysis

The association between LOH at 13q14.3 and CHC1-L expression was tested by the Fisher's exact test. Differences were judged significant at confidence levels greater than 95% (p<0.05). The non parametric Mann Whitney-U and Kruskal Wallis tests (StatView, Abacus Concepts, Inc., Berkeley, CA, 1996) were used to evaluate the variation in expression between prostate tissues.

RESULTS

a) Analysis of genomic DNA from prostate carcinomas.

36 sporadic prostate tumor DNAs and autologous normal DNAs for LOH status at 13q14.3 were analyzed, using the polymorphic markers D13S153, D13S272, and D13S284. All 36 prostate tumors were informative for at least one microsatellite marker.
LOH on at least one locus was found in 15 (42%) of the 36 tumor DNAs.

b) CHC1-L mRNA Steady-State Level

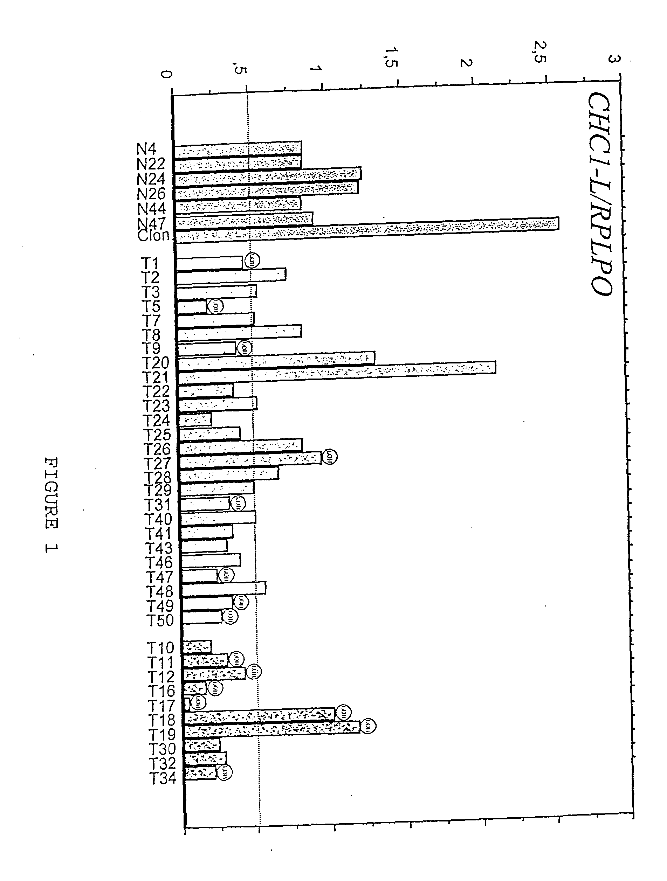

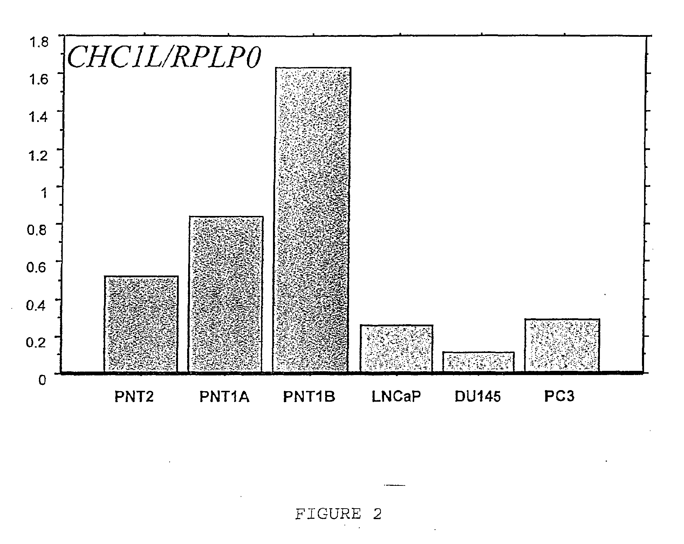

The relative expression levels of CHC1-L were quantified in 36 tumors (T), 6 matched normal prostate tissues (N) , and RNA from a pool of 47 normal human prostate tissues (Clontech). The expression levels are determined as ratios between CHC1-L and the reference gene RPLP0 to correct for variation in the amounts of RNA. As prostate samples contained mixtures of cell types, KRT18 a specific marker of epithelial cells, was also used as reference to quantify the expression levels of CHC1-L.
After duplicate assessments, the $N_{CHC1-L}$ value, calculated as described below, has been normalized such that the mean ratio of the 6 matched normal prostate samples equals 1.00 [Standard Deviation (SD) 0.21].
CHC1-L expression was significantly reduced (p=0.0002) in prostate tumors compared to normal prostate tissues when RPLPO was used for normalization (Figure 1). Moreover, there is a trend toward reduced expression of CHC1-L mRNA in androgen independent when compared to clinically localized tumors (p=0.058).
CHC1-L mRNA levels were at least 3-fold lower than normal prostate tissues in 8 clinically localized tumors (T5, T24, T31, T41, T43, T47, T49, and T50) and 7 hormone refractory tumors (T10, T11, T16, T17, T30, T32, and T34), and at least 2-fold lower in 5 (T1, T9, T22, T25, and T46) and 1 (T12), respectively. This apparent down regulation was confirmed when KRT18 was used for normalization.
It is noteworthy that CHC1-L mRNA levels were at least 1.5-fold lower in 6 clinically localized tumors (T3, T7, T23, T29, T40, and T48). One clinically localized tumor (T21) showed an increase in CHC1-L expression level.
The results are illustrated in Figure 1.
The three prostate tumor cell lines (LNCaP, DU145, and PC3) showed markedly decreased expression of CHC1-L compared to the prostatic epithelial cell lines (PNT2, PNT1A, and PNT1B) (p=0.049) (Figure 2).

c) Association between LOH status at 13q14.3 and CHC1-L expression.

In consideration of the potential effect of the loss of 13q14.3 locus on CHC1-L expression in prostate tumors, expression was considered to be reduced when the level of CHC1-L mRNA in the tumor was at least 2 fold lower (mean level in the normal prostate samples - 2.5 SD) than the mean level in the normal prostate tissues. Under this hypothesis, there is a trend toward a positive association between CHC1-L underexpression and LOH status at 13q14.3 (p=0.058). Twelve of the 15-13q14.3 deleted tumors showed an at least 2-fold decreased CHC1-L expression, whereas 12 of the 15 tumors that showed "normal" CHC1-L expression showed allelic retention at this locus.
The results are summarized in the following table 2.

TABLE 2

| CHC1-L underexpression | LOH at 13q14.3 | | |
|---|---|---|---|
| | yes (n=15) | no (n=21) | |
| yes (n=21) | 12 | 9 | p=0,058 |
| no (n=15) | 3 | 12 | |

d) CHC1-L variants in prostate tissues

PCR yielded a major CHC1-L-specific product of full length (325bp) in each sample. In addition, 3 additional bands of 65bp, 86bp, and 151bp shorter than the full length product were observed. Sequence analysis of these 260bp, 239bp, and 174bp products revealed the previously described CHC1-L variants (**11**) with a loss of exon 3, exon 2, and exon 2/3, respectively.

Co-amplification of all CHC1-L transcripts (spliced and wild-type) provided a semi-quantitative RT-PCR method in which the internal control was the co-amplified wild-type CHC1-L mRNA; comparative expression of spliced mRNA and wild-type CHC1-L mRNA could thus be determined for each sample. Three independent assays starting with different cDNAs generated from the same RNA sample were performed to ensure a reproducible frequency of alternatively spliced CHC1-L transcripts in each sample. The frequency of wild type CHC1-L variants is lower in the tumors than in the normal prostate specimen (64% *vs* 71 %; p=0.003), similar for D2 and D2/3-CHC1-L (2% *vs* 2%, and 16% *vs* 15%, respectively) and higher for D3 (18% *vs* 12%; p=0.007). Overall, the frequency of the 551aa isoform is reduced in tumors compared to normal prostate tissues (66% *vs* 73%).

To confirm and quantify these CHC1-L splice variants PCR was carried out using primer pairs 69U/191L, 90U/302L, 186U/383L, and 88U/381L which revealed wild-type, and CHC1-L splice variants generated by the fusion of exons 1 and 3, 2 and 4, and 1 and 4, respectively.

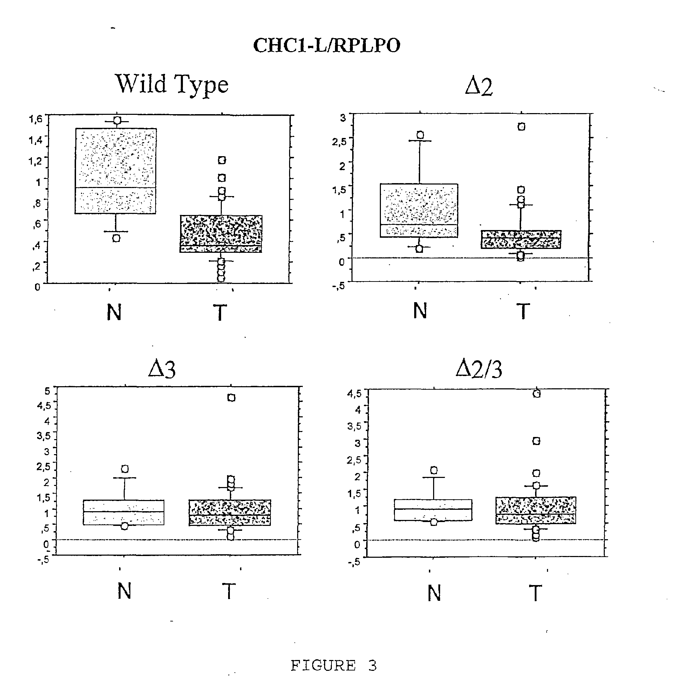

There is a significant decrease in wild type and D2-CHC1-L variant expression in prostate tumors compared to normal prostate tissues (p=0,001 and p=0,002, respectively). There is no significant difference in D3 and D2/3-CHC1-L variant expression between tumor and normal prostate tissues (Figure 3). Similarly, there is a significant decrease in wild type CHC1-L variant expression in prostate cancer cell lines compared to prostatic epithelial cell lines.

e) Association between LOH at 13q14.3, CHC1-L expression and Grade and Stage of prostate tumor.

Allelic loss at 13q14.3 and CHC1-L expression was analyzed according to tumor stage and grade. A trend was found towards both a higher frequency of LOH and decreased CHC1-L expression in hormone refractory tumors. Eight (31%) of the 26 patients with clinically localized prostate tumors showed allelic loss at this locus (3 stage pT2 and 5 stage pT3), while LOH was observed in 7 (70%) of the 10 hormone refractory tumors. Thirteen (50%) of the 26 patients with clinically localized prostate tumors showed a down regulation in CHC1-L compared to 8 (80%) of the 10 with hormone refractory tumors

There was no association between 13q loss or CHC1-L expression and the combined Gleason score (tumor grade).

REFERENCES

1.   PARKER SL., TONG T., BOLDEN S. & WINGO PA. ; CA Cancer J. Clin., 47, 5-27 (1997)

2.   LATIL A. & LIDEREAU R. ; Virchows Archiv, 432, 389-406 (1998)

3.   COONEY KA., WETZEL JC., MERAJVER SD., MACOSKA JA., SINGLETON TP. & WOJNO KJ. ; Cancer Res., 56, 1142-1145 (1996)

4.   HYYTINEN E-R., FRIERSON Jr HF., BOYD JC, CHUNG LWK. & DONG J-T., Gene Chrom., and Cancer, 25, 108-114 (1999)

5.   MELAMED J., EINHORN JM. & ITTMANN N., Clin. Cancer Res., 3, 1867-1872 (1997)

6.   AFONSO A., EMMERT-BUCK MR., DURAY PH., BOSTWICK DG., LINEHAN WM. & VOCKE CD., J. Urol., 162, 922-926 (1999)

7.   LATIL A., BIECHE I., PESCHE S., VOLANT A., VALERI A., FOURNIER G., CUSSENOT O. & LIDEREAU R., Human Pathol., 30, 809-815 (1999)

8.   UEDA T., EMI M., SUZUKI H., KOMIYA A., AKAKURA K., ICHIKAWA T., WATANABE M., SHIRAISHI T., MASAI T., IGARASHI T. & ITO H., Genes, Chrom. And Cancer, 24, 183-190 (1999)

9.   YIN Z., SPITZ MR., BABAIAN RJ., STROM SS., TRONCOSO P & KAGAN J., Oncogene, 18, 7576-7583 (1999)

10.  DODSON MK., CLIBY WB., XU H-J., DELACEY KA., HU S-X., KEENEY GL., LI J., PODRATZ KC., JENKINS RB. & BENEDICT WF., Cancer Res., 54, 610-613 (1994)

11.  YOO GH., XU HJ., BRENNAN JA., WESTRA W., HRUBAN RH., KOCH W, BENEDICT WF & SIDRANSKY D., Cancer Res., 54, 4603-4606 (1994)

12.  PEI L., MELMED S., SCHEITHAUER B., KOVACS K., BENEDICT WF & PRAGER D., Cancer Res., 55, 1613-1616 (1995)

13.  SEKI T., HAYASHI N. & NISHIMOTO T., J. Biochem. (Tokyo), 120, 207-214 (1996)

14.  LI C., LARSSON C., FUTREAL A., LANCASTER J;, PHELAN C;, ASPENBLAD U., SUNDELIN B., LIU Y., EKMAN P, AUER G & BERGERHEIM USR., Oncogene, 16, 481-487 (1998)

15.  DEVILDER M-C., CADORET E., CHEREL M., MOREAU I., RONDEAU G., BEZIEAU S. & MOISAN J-P., Genomics, 54, 99-106 (1998)

16.  OHTSUBO M., KAI R., FURUNO N., SEKIGUCHI T., HAYASHIDA H., KUMA K., MIYATA T., FUKUSHIGE S., MUROTSU T., OKAZAKI H. & NISHIMOTO T., Genes Dev., 1, 585-593 (1987)

17.  KLEBE C., PRINTZ H., WITTINGHOFER A. & GOODY RS., Biochemistry, 34, 12543-12552 (1995)

18.  MELLINGER GT., GLEASON D. & BAILAR JIII, J. Urol., 97, 331-337 (1967)

19.  SCHRODER FH., HERMANEK P., DENIS L., FAIR WR., GOSPODAROWICZ MK. & PAVONE-MACALUSO M., Prostate Suppl., 4, 129-138 (1992)

20.  BERTHON P., CUSSENOT O., HOPWOOD L., LE DUC A. & MAITLAND NJ., Int. J. Oncol., 6, 333-343 (1995)

21.  CHOMCZYNSKI P. & SACCHI N., Anal. Biochem., 162, 156-159 (1987)

22.  DIB C., FAURE S., FIZAMES C., SAMSON D., DROUOT N., VIGNAL A., MILLASSEAU P., MARC S., HAZAN J., SEBOUN E., LATHROP M., GYAPAY G;, MORISSETTE J & WEISSENBACH J., Nature, 380, 152-154 (1996)

23.  McGROGAN D., LEVY A., BOSTWICK D., WAGNER M., WELLS D. & BOOKSTEIN R., Genes Chrom; Cancer, 10, 151-159 (1994)

24. ALTSCHUL SF., GISH W., MILLER W., MYERS EW. & LIPMAN DJ., J. Mol. Biol., 215, 403-410 (1990)

**Claims**

1. Polypeptide, of about 15 to about 30 amino acids, corresponding to the protein fragment encoded by the 5' end of the tumor suppressor gene CHC1-L.

2. Polypeptide according to claim 1, corresponding to the whole, or a part of the, sequence SEQ ID NO : 1.

3. Polypeptide according to claim 1 or 2, **characterized in that** it shows one or more conservative(s) substitution(s) and/or addition(s) and/or deletion(s) of one or more amino acid(s).

4. Polypeptide according to any one of claims 1 to 3, **characterized in that** it is substituted at its N-terminal and/or C-terminal end(s).

5. Polypeptide according to claim 4, **characterized in that** the substituant is an active principle selected in the group consisting in a growth-factor, a neuropeptide, a transcription factor, a signal molecule, a repressor or antagonist of transcription or replication, a toxin, an antitoxin or an apoptosis inducer.

6. Nucleotide sequence encoding for a polypeptide according to any one of claims 1 to 5.

7. Nucleotide sequence, **characterized in that** said sequence shows the whole, or a part of the, sequence SEQ ID NO : 2.

8. Recombinant vector, **characterized in that** it comprises, or includes, at least the nucleotide sequence according to claim 6 or 7.

9. Vector according to claim 8, **characterized in that** it consists in a viral vector.

10. Vector according to claim 8, **characterized in that** it consists in a non viral vector.

11. Vector according to claim 10, **characterized in that** it consists in a plasmid possibly covered with a synthetic polymer or a polysaccharide, a protein, a peptide fragment or a mixture of two or more of the above.

12. Transfected cell, **characterized in that** it includes a nucleotide sequence according to claim 6 or 7 and/or a vector according to any one of claims 8 to 11.

13. Use of a polypeptide according to any one of claims 1 to 5 and/or a nucleotide sequence according to claim 6 or 7 and/or a vector according to any one of claims 8 to 11 for the preparation of a pharmaceutical composition.

14. Pharmaceutical composition including, in combination with one or more pharmaceutically acceptable carrier(s), a polypeptide according to any one of claims 1 to 5 and/or a nucleotide sequence according to claim 6 or 7 and/or a vector according to any one of claims 8 to 11.

15. Pharmaceutical composition including, in combination with one or more pharmaceutically acceptable carrier(s), a factor mimicking the function of the polypeptide according to any one of claims 1 to 5 and/or nucleotide sequence according to claim 6 or 7 and/or vector according to any one of claims 8 to 11.

16. Pharmaceutical composition according to claim 14 or 15 for cancer treatment.

17. Pharmaceutical composition according to claim 16 for prostate cancer treatment.

18. Use of a polypeptide according to any one of claims 1 to 5 and/or a nucleotide sequence according to claim 6 or 7 and/or a vector according to any one of claims 8 to 11 as an *in vitro* diagnostic agent.

19. Method for the *in vitro* diagnosis of the prostate cancer consisting in evaluating the RNA level of the peptide according to any one of claims 1 to 5 by measuring the ratio between the long form (L) and the short form (S) of CHC1-L.

**20.** Method according to claim 19, **characterized in that** it consists in :

    a) measuring the level of RNA encoding for the long form (L) of CHC1-L,
    b) measuring the level of RNA encoding for the short form (S) of CHC1-L,
    c) determining the ratio L/S, and
    d) declaring the diagnosis positive for cancer if the obtained ratio of step c) is close to 2.

**21.** Kit for the implementation of the process according to claim 19 or 20 including :

    a) primer(s) for the amplification of the RNA encoding for the two forms (L) and (S) of CHC1-L,
    b) polymorphic markers,
    c) primer(s) for the PCR amplification of the polymorphic markers, and
    d) buffers and enzymes for the amplification, marking and hybridization reactions.

EP 1 201 680 A2

FIGURE 1

FIGURE 2

## CHC1-L/RPLPO

FIGURE 3